# EUROPEAN PATENT APPLICATION

(11) **EP 4 461 807 A1**
(43) Date of publication of application: **13.11.2024**
(21) Application number: 23740320.9
(22) Date of filing: 13.01.2023
(51) Int. Cl.: C12N 5/077, A61K 35/34, A61L 27/38, A61L 27/44, A61P 1/16, A61P 35/00, C12N 15/09

(54) **CELL CULTURE SHEET FOR TREATING LIVER DYSFUNCTION**

(30) Priority: 14.01.2022 JP 2022004091
(71) Applicant: OSAKA UNIVERSITY, Suita-shi Osaka 565-0871 (JP); Terumo Kabushiki Kaisha, Tokyo 151-0072 (JP)
(72) Inventor: EGUCHI, Hidetoshi, Suita-shi, Osaka 565-0871 (JP); TOMIMARU, Yoshito, Suita-shi, Osaka 565-0871 (JP); MIYAGAWA, Shigeru, Suita-shi, Osaka 565-0871 (JP); TOYA, Keisuke, Suita-shi, Osaka 565-0871 (JP); OHASHI, Fumiya, Ashigarakami-gun, Kanagawa 259-0151 (JP)
(74) Representative: Prüfer & Partner mbB Patentanwälte · Rechtsanwälte
(86) International application number: PCT/JP2023/000697
(87) International publication number: WO 2023/136312

(57) **Abstract**

[Problem]

It is an object of the present invention to provide a means for treating liver dysfunction.

[Solution]

The above object was achieved by providing a sheet-shaped cell culture containing skeletal myoblasts for treating liver dysfunction or improving liver function; a method for producing the sheet-shaped cell culture, including a step of seeding a cell population containing skeletal myoblasts on a culture substrate, a step of forming a sheet of the cell population into a sheet to form a sheet-shaped cell culture, and a step of detaching the formed sheet-shaped cell culture from the culture substrate; and a method for treating liver dysfunction, including a step of applying the sheet-shaped cell culture to a site exhibiting the liver dysfunction.

## Description

### Technical Field

The present invention relates to a sheet-shaped cell culture and others for treating liver dysfunction.

### Background Art

Recently, attempts have been made to implant various types of cells for repairing damaged tissues, etc. For example, for repairing myocardial tissue damaged by ischemic heart diseases such as angina pectoris and myocardial infarction, attempts have been made to use, e.g., fetal cardiomyocytes, skeletal myoblasts, mesenchymal stem cells, cardiac stem cells, ES cells and iPS cells (Non Patent Literature 1).

As part of the attempts, a cell construct formed on a scaffold and a sheet-shaped cell culture, which is a sheet formed of cells, have been developed (Patent Literature 1, Non Patent Literature 2).

For the application of the sheet-shaped cell culture to treatment, for example, studies have been made on use of an epidermal sheet-shaped cell culture for treating skin damages caused by, e.g., burn; use of a corneal epithelial sheet-shaped cell culture for corneal damage; and use of an oral mucosa sheet-shaped cell culture for esophageal cancer endoscopic resection. Some of them have entered the stage of clinical application.

As one of the applications, it has been proposed to use a sheet-shaped cell culture for treating damage to organs such as digestive tract. For example, Patent Literature 2 discloses the use of a sheet-shaped cell culture containing mesenchymal stem cells for treating or preventing leakage from a damaged portion of the gastrointestinal tract caused by suture failure, etc.

Furthermore, Patent Literature 3 discloses an engraftment sheet material having satisfactory engraftment efficiency to the surface of an organ and satisfactory operability in clinical applications. More specifically, it has been demonstrated that a myoblast sheet, which has an extracellular matrix layer on one of the surfaces and a biodegradable gel layer on the other surface, can be satisfactorily engrafted in the surface of the liver or colon by implanting the sheet such that the surface the extracellular matrix layer faces the organ.

### Citation List

### Patent Literature

Patent Literature 1: JP 2007-528755 A
Patent Literature 2: WO 2017/130802
Patent Literature 3: WO 2021/149830

### Non Patent Literature

Non Patent Literature 1: Haraguchi Y. et al., Stem Cells Transl. Med., 1(2), 136-141 (2012)
Non Patent Literature 2: Sawa Y. et al., Surg. Today, 42(2), 181-184 (2012)

### Summary of Invention

### Technical Problem

It is an object of the present invention to provide a means for treating liver dysfunction.

### Solution to Problem

The present inventors have studied a method for treating liver dysfunction. During the studies, they focused on the possibility of treating liver dysfunction by cytokines produced from a sheet-shaped cell culture. Then, they conducted studies with a view to demonstrating the possibility. As a result, they found that proliferation of the liver cells, regeneration of the liver tissue and angiogenesis are promoted by using a sheet-shaped cell culture containing skeletal myoblasts, more specifically, cytokines produced from the sheet-shaped cell culture. Based on the finding, they conducted further studies, with the result that the present invention was accomplished.

More specifically, the present invention relates to the followings.
[1] A sheet-shaped cell culture containing skeletal myoblasts for treating liver dysfunction or improving liver function.
[2] The sheet-shaped cell culture according to [1], in which the liver dysfunction is hepatitis, liver fibrosis, liver cirrhosis, liver cancer, or liver failure.
[3] The sheet-shaped cell culture according to [1] or [2], in which the liver dysfunction is liver cirrhosis.
[4] The sheet-shaped cell culture according to [1] or [2], in which the liver dysfunction is liver cancer.
[5] The sheet-shaped cell culture according to [4], for application to the liver after liver cancer resection.
[6] The sheet-shaped cell culture according to any one of [1] to [5], for promoting proliferation of the liver cells, regeneration of the liver tissue and/or angiogenesis.
[7] The sheet-shaped cell culture according to any one of [1] to [6], for suppressing liver fibrosis.
[8] The sheet-shaped cell culture according to any one of [1] to [7], for treating liver dysfunction through cytokine production.
[9] The sheet-shaped cell culture according to any one of [1] to [8], further containing a reinforcement layer containing a gel and/or a polymer.
[10] A method for producing the sheet-shaped cell culture according to any one of [1] to [9], including a step of seeding a cell population containing skeletal myoblasts on a culture substrate; a step of forming a sheet of the cell population seeded to form a sheet-shaped cell culture; and a step of detaching the formed sheet-shaped cell culture from the culture substrate.
[11] A method for treating liver dysfunction, including a step of applying the sheet-shaped cell culture according to any one of [1] to [9] to a site exhibiting the liver dysfunction.
[12] The method according to [11], in which the step of applying the sheet-shaped cell culture to a site exhibiting the liver dysfunction is performed by implanting the sheet-shaped cell culture to a liver surface by an implantation device.

### Advantageous Effects of Invention

According to the present invention, it is possible to treat liver dysfunction such as hepatitis, liver fibrosis, liver cirrhosis, liver cancer, or liver failure. In particular, according to the present invention, it is possible to treat terminal-stage decompensated cirrhosis whose improvement cannot be expected by current internal therapy as well as to treat liver failure of residual liver after liver cancer resection. Furthermore, according to the present invention, it is possible to promote proliferation of the liver cells, regeneration of the liver tissue and/or angiogenesis. Furthermore, according to the present invention, it is possible to suppress liver fibrosis. Moreover, according to the present invention, it is possible to treat liver dysfunction through cytokine production.

### Brief Description of Drawings

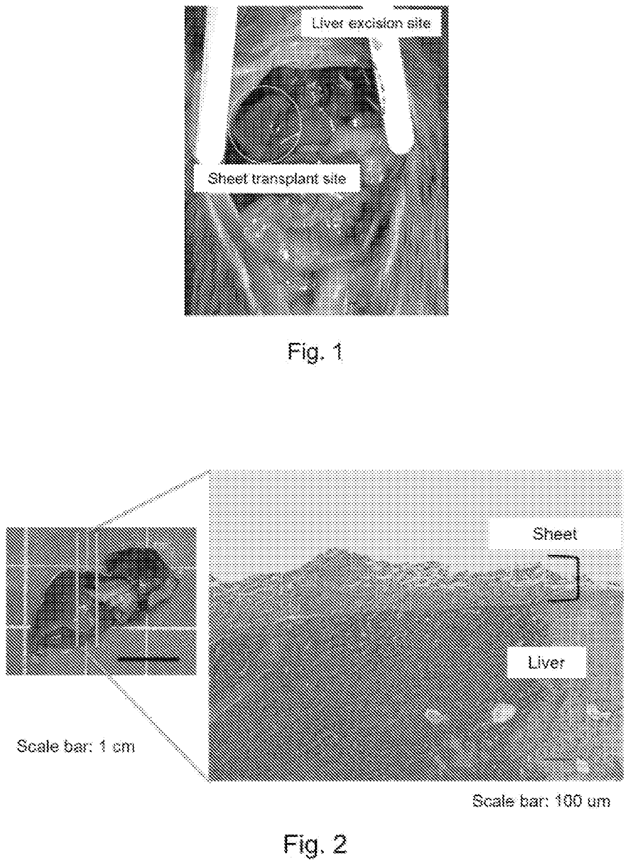

Fig. 1 shows the liver after partial hepatectomy and having a sheet-shaped cell culture implanted therein in a 70% partial hepatectomy model mouse. The solid-line circle shows an implant site of a sheet-shaped cell culture and the dashed-line circle shows a partial hepatectomy site.
Fig. 2 shows a hematoxylin and eosin (HE)-stained image of a section of a sheet-shaped cell culture implantation site in a 70% partial hepatectomy model mouse.

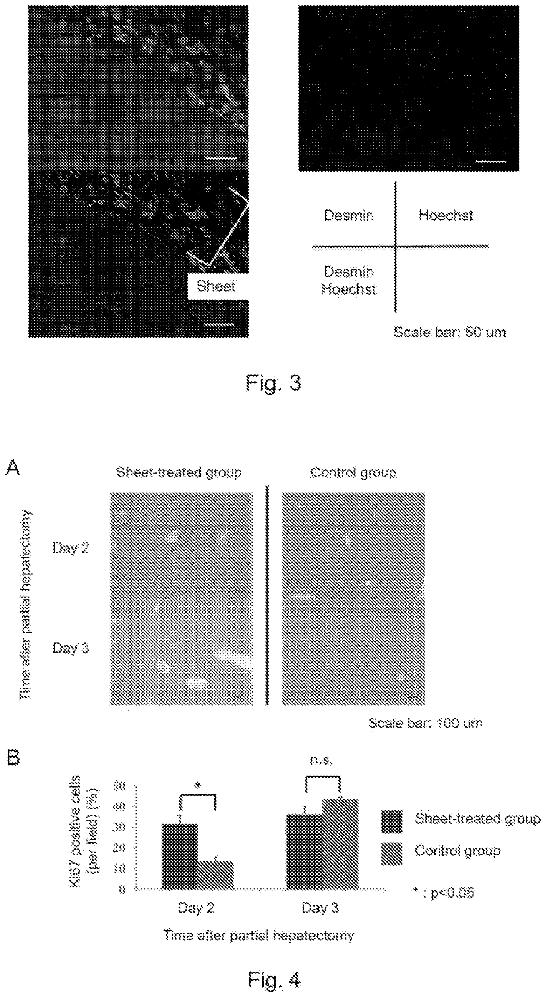

Fig. 3 shows an immunofluorescence stained image of a section of a sheet-shaped cell culture implantation site in a 70% partial hepatectomy model mouse.
Fig. 4 shows the result of immunostaining of a section of an implantation site of a sheet-shaped cell culture in a 70% partial resection model mouse. Fig. 4A shows immunostained images of sections of a sheet-shaped cell culture implantation site. Fig. 4B shows a graph prepared by counting the number of Ki67-positive cells per field of view and plotting the counts. Each individual value is an average value in a sheet-treated group (n = 7) and a control group (n = 12) and an error bar represents a standard deviation.

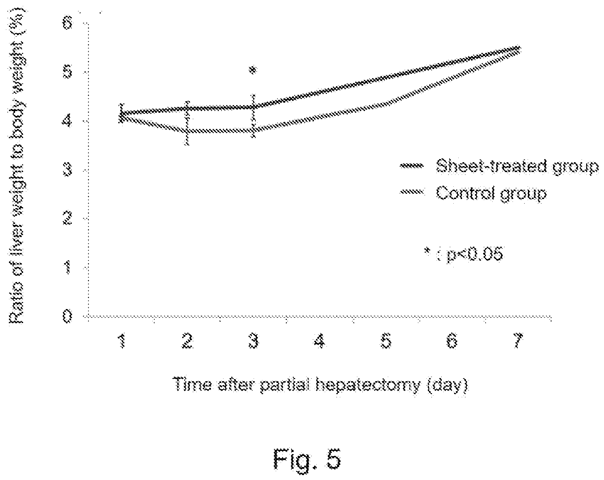

Fig. 5 shows a change of the liver-weight/body-weight ratio in 70% partial-hepatectomy model mice with the lapse of time after surgery. Each individual value is an average value in a sheet-treated group (n = 7) and a control group (n = 12) and an error bar represents a standard deviation.

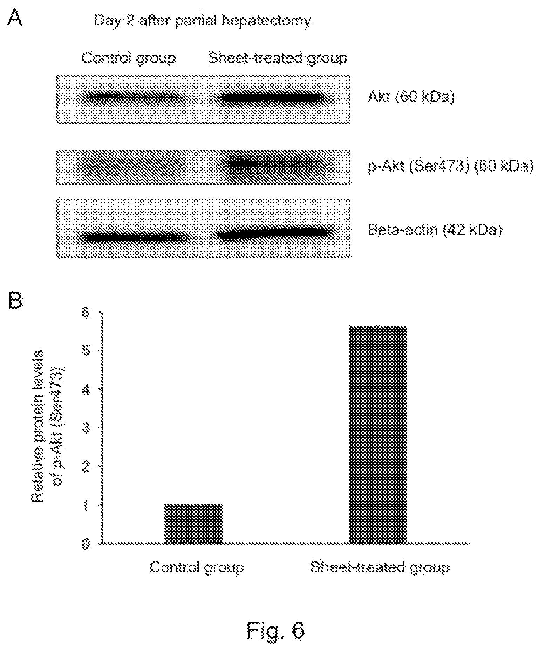

Fig. 6 shows the results of western blotting of lysates of sheet-shaped cell culture implanted sites in 70% partial-hepatectomy model mice with Akt, p-Akt (Ser473), and β-actin, respectively. Fig. 6A shows the images obtained and Fig. 6B shows a graph prepared by quantifying band regions of p-Akt (Ser473) and plotting the quantified values. Each individual value is an average value in a sheet-treated group (n = 7) and a control group (n = 12) and an error bar represents a standard deviation.

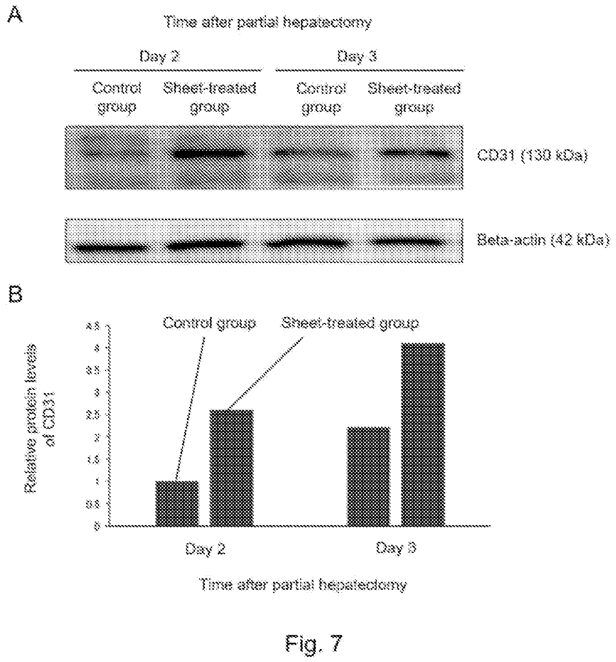

Fig. 7 shows the results of western blotting of lysates of sheet-shaped cell culture implanted sites in 70% partial-hepatectomy model mice with CD31 and β-actin. Fig. 7A shows the images obtained and Fig. 7B shows graphs prepared by quantifying band regions of CD31 and plotting the quantified values. Each individual value is an average value in a sheet-treated group (n = 5) and a control group (n = 5) and an error bar represents a standard deviation.

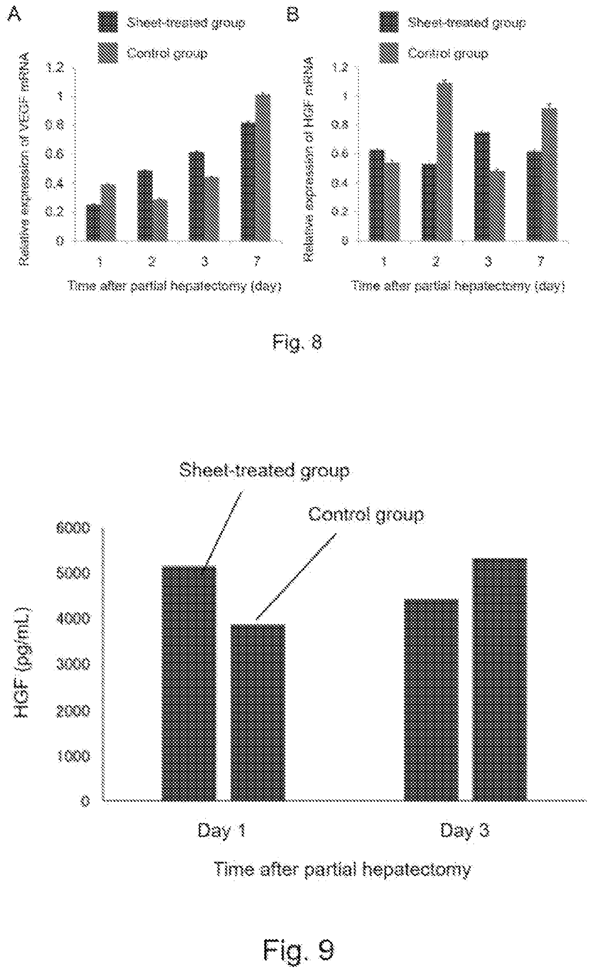

Fig. 8 shows the results of real-time PCR of RNA extracted from lysates of sheet-shaped cell culture implanted sites in 70% partial-hepatectomy model mice. Fig. 8A shows the mRNA expression level of VEGF, and Fig. 8B shows the mRNA expression level of HGF. Each individual value is an average value in a sheet-treated group (n = 7) and a control group (n = 12) and an error bar represents a standard deviation.
Fig. 9 shows the results of ELISA of lysates of sheet-shaped cell culture implanted sites in 70% partial-hepatectomy model mice with HGF. Each individual value is an average value in a sheet-treated group (n = 7) and a control group (n = 12) and an error bar represents a standard deviation.

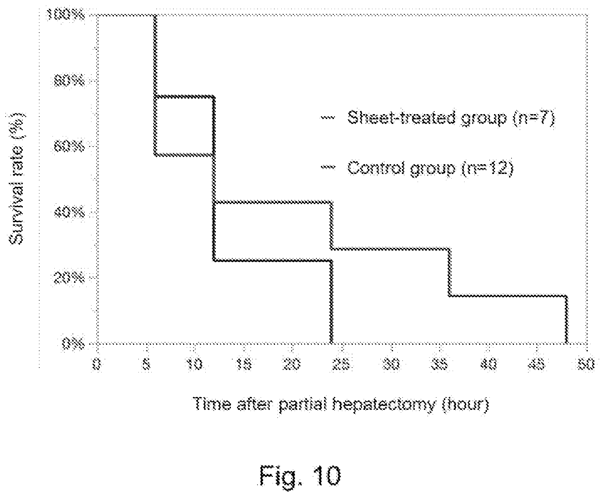

Fig. 10 a change in survival rate of 90% partial-hepatectomy model mice in a sheet-treated group (n = 7) and a control group (n = 12) with the lapse of time after surgery.

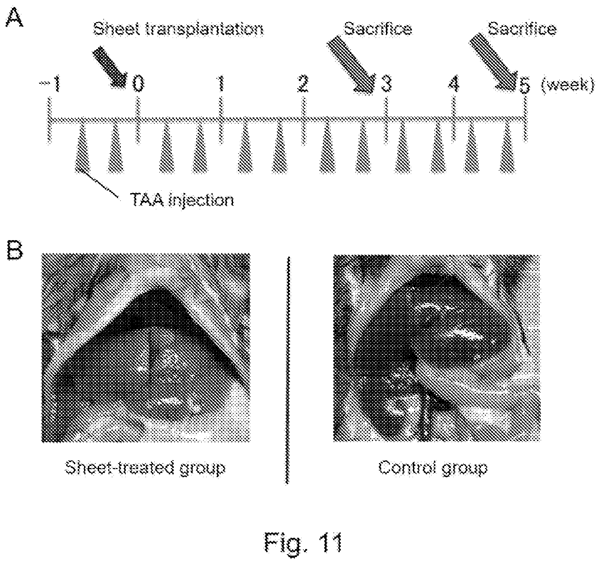

Fig. 11 shows a method for preparing and treating liver-fibrosis model mice. Fig. 11A shows the schedule listing injection of thioacetamide (TAA), treatment with a sheet-shaped cell culture and sacrificial death. Fig. 11B shows the liver (sheet-treated group) implanted with a sheet-shaped cell culture and the liver (control group) not implanted with a sheet-shaped cell culture.

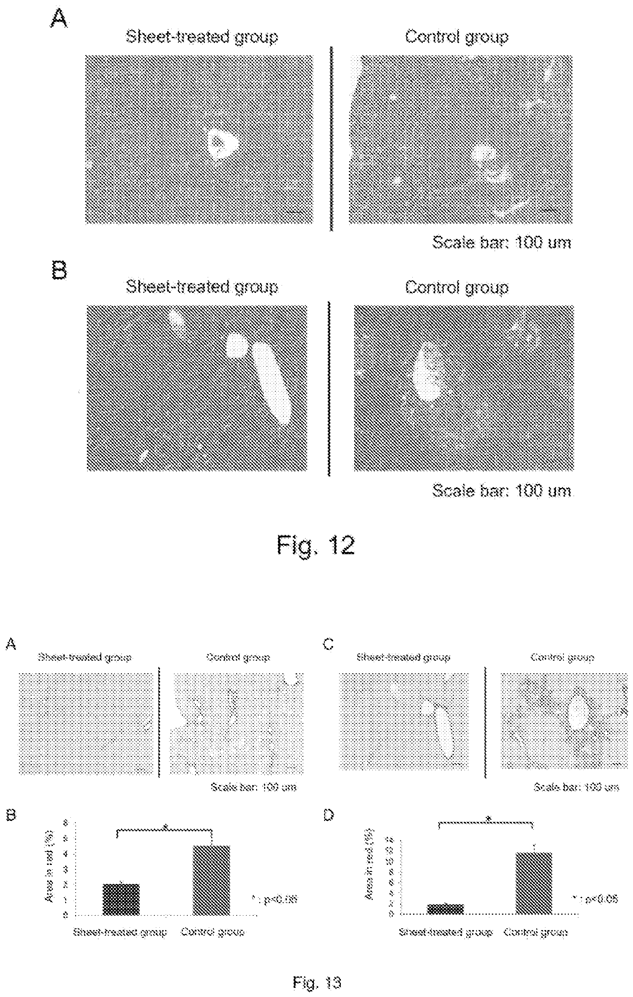

Fig. 12 shows hematoxylin and eosin (HE)-stained images of liver sections of liver-fibrosis model mice (Fig. 12A: 3 weeks after surgery, Fig. 12B: 5 weeks after surgery).
Fig. 13 shows Sirius red-stained images of liver sections of liver-fibrosis model mice. Figs. 13A and C show Sirius red-stained images of liver sections (Fig. 13A: 3 weeks after surgery, Fig. 13C: 5 weeks after surgery). Figs. 13B and D show graphs prepared by quantifying red regions and plotting the quantified values (Fig. 13B: 3 weeks after surgery, Fig. 13D: 5 weeks after surgery). Each individual value is an average value in a sheet-treated group (n = 5) and a control group (n = 5) and an error bar represents a standard deviation.

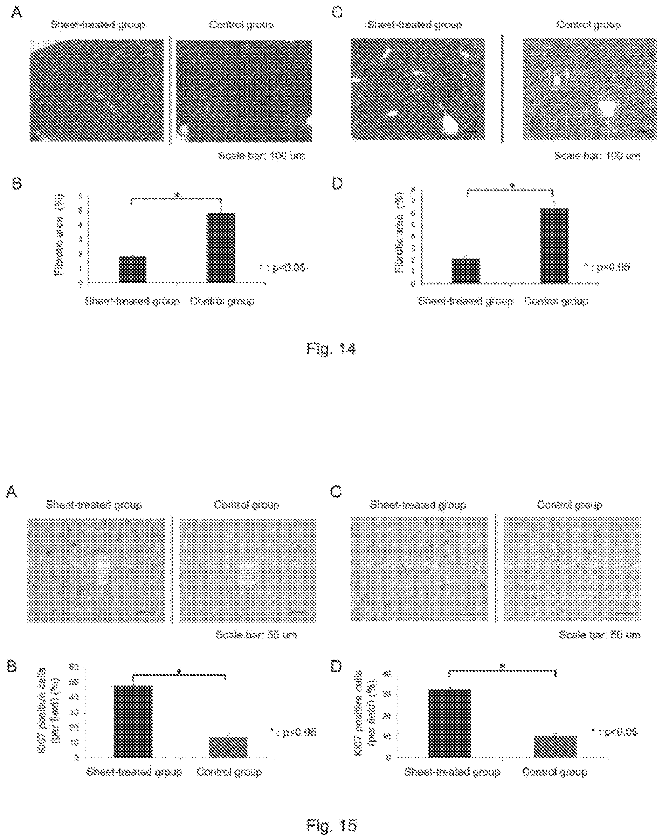

Fig. 14 shows the Masson's trichrome staining results of liver sections of liver-fibrosis model mice. Figs. 14A and C show the Masson's trichrome stained images of liver sections (Fig. 14A: 3 weeks after surgery, Fig. 14C: 5 weeks after surgery). Figs. 14B and D show graphs prepared by quantifying fibrosis regions and plotting the quantified values (Fig. 14B: 3 weeks after surgery, Fig. 14D: 5 weeks after surgery) Each individual value is an average value in a sheet-treated group (n = 5) and a control group (n = 5) and an error bar represents a standard deviation.
Fig. 15 shows immunostaining results of liver sections of liver-fibrosis model mice. Figs. 15A and C show immunostained images of the liver sections (Fig. 15A: 3 weeks after surgery, Fig. 15C: 5 weeks after surgery). Figs. 15B and Dshow graphs prepared by counting the number of Ki67-positive cells per field of view and plotting the counts (Fig. 15B: 3 weeks after surgery, Fig. 15D: 5 weeks after surgery). Each individual value is an average value in a sheet-treated group (n = 5) and a control group (n = 5) and an error bar represents a standard deviation.

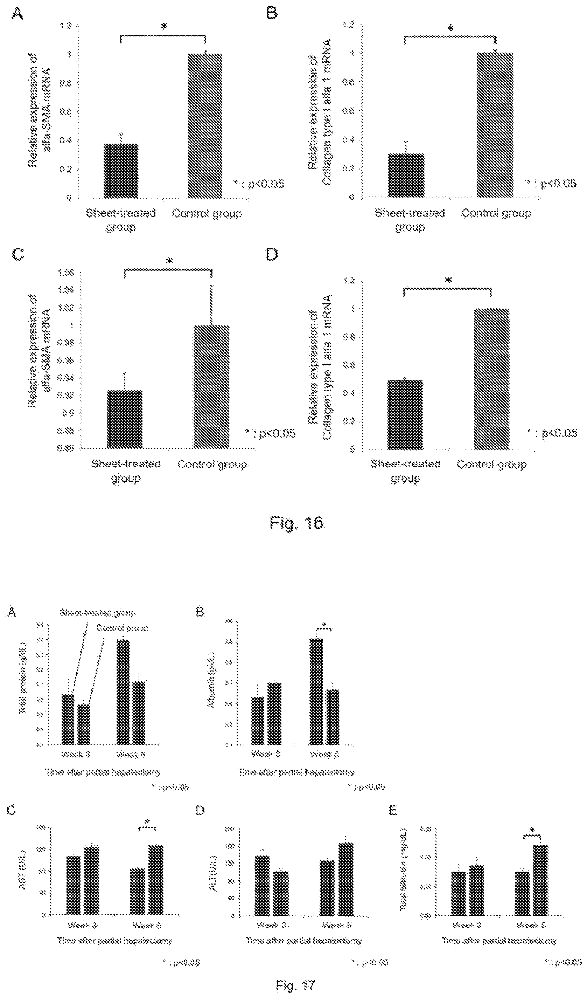

Fig. 16 shows the results of real-time PCR of RNAs extracted from liver lysates of liver-fibrosis model mice. Figs. 16A and C show the expression levels of αSMA mRNAs (Fig. 16A: 3 weeks after surgery, Fig. 16C: 5 weeks after surgery). Figs. 16B and D show the expression levels of collagen type I α1 mRNAs (Fig. 16B: 3 weeks after surgery, Fig. 16D: 5 weeks after surgery). Each individual value is an average value in a sheet-treated group (n = 5) and a control group (n = 5) and an error bar represents a standard deviation.
Fig. 17 shows the ELISA results of liver lysates of liver-fibrosis model mice. Figs. 17A to E show the expression levels of a total protein, albumin, aspartate aminotransferase (AST), alanine aminotransferase (ALT), and total bilirubin, respectively. Each individual value is an average value in a sheet-treated group (n = 5) and a control group (n = 5) and an error bar represents a standard deviation.

In the specification, unless otherwise specified, all technical terms and scientific terms used in the specification have the same meaning that those skilled in the art commonly understand. All patents, applications and other publications and information cited herein are incorporated in their entirety herein by reference.

### Description of Embodiments

### [Sheet-shaped cell culture for Treating Liver Dysfunction]

An aspect of the present invention relates to a sheet-shaped cell culture containing skeletal myoblasts for treating liver dysfunction or improving liver function (sometimes referred to as "the sheet-shaped cell culture of the present invention").

In an embodiment, liver dysfunction is hepatitis, liver fibrosis, liver cirrhosis, liver cancer, or liver failure.

In an embodiment, liver dysfunction is liver cirrhosis.

In an embodiment, liver dysfunction is liver cancer.

In an embodiment, the sheet-shaped cell culture of the present invention is used for application to the liver after liver cancer resection.

In an embodiment, the sheet-shaped cell culture of the present invention is used for promoting the proliferation of the liver cells, regeneration of the liver tissue and/or angiogenesis.

In an embodiment, the sheet-shaped cell culture of the present invention is used for suppressing liver fibrosis.

In an embodiment, the sheet-shaped cell culture of the present invention is used for treating liver dysfunction through cytokine production.

In an embodiment, the sheet-shaped cell culture of the present invention further contains a reinforcement layer containing a gel and/or a polymer.

The liver is an organ having a wide variety of functions such as metabolism, maintenance of body-fluid homeostasis, and digestion as well as regeneration ability. In the liver, blood flows in from the hepatic artery and portal vein, passes through the central vein and flows out from the hepatic vein. The liver tissue is constituted of a mass of liver lobules and the central vein passes through the central shaft part of a liver lobule. The liver cells are radially arranged around the central vein to form the liver cell plate and occupy most of the whole cells in the liver. In the specification, the "liver cells" are interchangeably used with "hepatic parenchymal cells". The cells other than the liver cells in the liver are called "liver non-parenchymal cells". Examples thereof include sinusoidal endothelial cells, Kupffer cells, dendritic cells, natural killer (NK) cells, hepatic stellate cells, and monocyte-derived macrophages.

In the present invention, the liver is not limited as long as it is the liver of a living organism. Examples of the liver include the liver of a human, a non-human primate, a rodent (such as a mouse, a rat, hamster or a guinea pig) and a mammal such as a rabbit, a dog, a cat, a pig, a horse, a cow, a goat or sheep.

In the present invention, the "liver function" refers to a function of a healthy liver such as metabolism, maintenance of body-fluid homeostasis and digestion.

In the present invention, the "liver dysfunction" refers to inhibition, suppression and/or impairment of the liver function due to some cause. For example, the liver dysfunction refers to hepatitis, liver fibrosis, liver cirrhosis, liver cancer, or liver failure.

In the present invention, the "healthy liver" refers to an unharmed (intact) liver not influenced by, e.g., a disease, a disorder and/or a treatment.

In the present invention, the term "skeletal myoblasts" refers to myoblasts present in the skeletal muscle. The skeletal myoblasts are commonly known in the technical field and can be prepared from the skeletal muscle by any one of the methods commonly known (for example, a method disclosed in JP 2007-89442) or is commercially available (for example, Lonza, Cat# CC-2580). Skeletal myoblasts may be identified by a maker. Examples of the marker include, but are not limited to, CD56, α7 integrin, myosin heavy chain IIa, myosin heavy chain IIb, myosin heavy chain IId (IIx), MyoD, Myf5, Myf6, myogenin, desmin and PAX3. In a specific embodiment, skeletal myoblasts are CD56 positive. Further, in a specific embodiment, skeletal myoblasts are CD56 positive and desmin positive. Skeletal myoblasts may be derived from any organism having skeletal muscle. Examples of the organism include, but are not limited to, a human, a non-human primate, a rodent (such as a mouse, a rat, a hamster or a guinea pig) and a mammal such as a rabbit, a dog, a cat, a pig, a horse, a cow, a goat or sheep. In an embodiment, the skeletal myoblasts refer to mammalian skeletal myoblasts. In a specific embodiment, the skeletal myoblasts refer to human skeletal myoblasts. Furthermore, the skeletal myoblasts can be collected from any skeletal muscle. In an embodiment, skeletal myoblasts refer to skeletal myoblasts derived from the thigh, neck or abdomen.

In the present invention, the "sheet-shaped cell culture" refers to a sheet formed of cells connected to each other. Cells may be mutually connected directly (including cells connected via a cellular element such as an adhesion molecule) and/or via an intervening substance. The intervening substance is not particularly limited as long as it can connect cells at least physically (mechanically). Examples thereof include an extracellular matrix. The intervening substance is preferably a cell-derived substance and particularly a substance derived from cells constituting a cell culture. Cells are at least physically (mechanically) connected and further may functionally, for example, chemically or electrically connected. The sheet-shaped cell culture may be composed of a single cell layer (uni-layer), two or more cell layers (a laminate (multilayer), for example, 2 layers, 3 layers, 4 layers, 5 layers or 6 layers). Furthermore, the sheet-shaped cell culture may not have a clear layer-structure of cells but has a three-dimensional structure having a thickness beyond the thickness of a single cell. For example, cells may not be uniformly arranged in the horizontal direction, that is, may be non-uniformly arranged in the vertical section of a sheet-shaped cell culture.

The sheet-shaped cell culture of the present invention is applied to the liver to treat liver dysfunction or improve liver function. In an embodiment, the sheet-shaped cell culture of the present invention is applied to the liver surface to treat liver dysfunction or improve liver function. In an embodiment, the sheet-shaped cell culture of the present invention treats liver dysfunction or improves liver function by applying it to the liver surface by an implantation device. In an embodiment, the sheet-shaped cell culture of the present invention treats liver dysfunction or improves liver function by applying it to the liver surface by an implantation device.

In the present invention, the "surface of the liver" refers to any surface of a healthy liver or liver influenced in some way by, e.g., a disease, a disorder, and/or a treatment. In an embodiment, the liver surface is the surface of the liver after partial hepatectomy. In an embodiment, the liver surface is the surface (having a fibrosis region) of the liver exhibiting liver fibrosis.

In the present invention, the "application" refers to attaching implanting, and/or engrafting a sheet-shaped cell culture.

In the present invention, the "implantation device" refers to a medical device capable of applying the sheet-shaped cell culture to the liver. In an embodiment, the implantation device is a medical device capable of applying the sheet-shaped cell culture to the liver surface. The implantation device is, for example, a medical device having a planar structure capable of supporting and detaching a sheet-shaped cell culture. In view of operating it within the abdominal cavity, the implantation device preferably has a form that can be inserted and passed through a cylindrical body which has been inserted in a body cavity in an endoscopic surgery.

In the present invention, "hepatitis" refers to an inflammation in the liver. Examples of hepatitis include viral hepatitis (such as hepatitis A, B and C), alcoholic hepatitis, nonalcoholic steatohepatitis, drug-induced hepatitis and autoimmune hepatitis. Hepatitis is roughly divided into acute hepatitis and chronic hepatitis. Acute hepatitis is hepatitis that temporally occurs, whereas chronic hepatitis is hepatitis lasting for a long time, with the result that, e.g., a fibrotic change of the liver, and degeneration and necrosis of liver cells, may occur. Chronic hepatitis develops, through, e.g., a fibrotic change of the liver and degeneration and necrosis of liver cells, into liver fibrosis and liver cirrhosis, and then, the liver cancer.

In the present invention, "liver fibrosis" refers to the formation of a scar tissue in the liver, which is developed by replacing the parenchymal tissue of a healthy liver for connective tissue by a fibrotic change of the liver, and degeneration and necrosis of liver cells caused by repetitive damage and chronic hepatitis, followed by accumulating an extracellular matrix component such as collagen.

In the present invention, "liver cirrhosis" refers to the formation of a large amount of scar tissue in the liver, which is developed by replacing the parenchymal tissue of a healthy liver for a large amount of connective tissue by a fibrotic change of the liver, and degeneration and necrosis of liver cells caused by repetitive damage and chronic hepatitis followed by accumulating a large amount of an extracellular matrix component such as collagen. Liver cirrhosis in the initial stage having no symptoms is called compensated cirrhosis (a failure caused in part of the liver can be compensated by the remaining part). In contrast, liver cirrhosis in the terminal stage having a symptom such as jaundice, spider hemangioma and palmar erythema, which appears as a disease state progresses, is called decompensated cirrhosis. Compensated cirrhosis and initial-stage decompensated cirrhosis can be treated by current internal therapy (e.g., interferon, glycyrrhizin preparation, diuretic, albumin preparation and diet remedy). In contrast, in the terminal-stage decompensated cirrhosis, no improvement thereof is expected by current internal therapy. After the disease progresses into liver cancer and liver failure, there is no choice but a surgical treatment.

In an embodiment, the sheet-shaped cell culture of the present invention is used for treating compensated cirrhosis or decompensated cirrhosis. In an embodiment, the sheet-shaped cell culture of the present invention is used for treating an initial- or terminal-stage decompensated cirrhosis.

In the present invention, "liver cancer" refers to a malignant tumor arising in the liver. Liver cancer is roughly divided into primary liver cancer and metastatic liver cancer. The primary liver cancer is mostly hepatocellular carcinoma (malignant tumor derived from liver cells). Hepatocellular carcinoma often results from progression to liver cancer through chronic hepatitis, liver fibrosis, liver cirrhosis.

In the present invention, "liver failure" refers to a significant reduction in the function of healthy liver, such as metabolism, body-fluid homeostasis and digestion, due to damage to most of the liver. Examples of the cause for liver failure include viral hepatitis, liver cirrhosis, alcoholic liver injury and drug-induced liver injury. Furthermore, the residual liver after liver cancer resection sometimes shows liver failure. Liver failure is roughly classified into acute liver failure and chronic liver failure. Acute liver failure quickly progresses in a short period of time, whereas chronic liver failure gradually progresses over a long period of time.

In the present invention, "applying to the liver after resection of liver cancer" refers to applying, implanting, and/or engrafting the sheet-shaped cell culture of the present invention in the liver remaining after partial or complete resection of liver cancer. In an embodiment, the sheet-shaped cell culture of the present invention is applied to the surface of the residual liver after liver cancer is partially or completely removed. In an embodiment, the sheet-shaped cell culture of the present invention is applied to the surface of the residual liver after liver cancer is partially or completely removed by implantation device. In an embodiment, the liver after liver cancer resection shows liver failure.

In the present invention, "liver cells" refer to cells radially arranged around the central vein of a liver lobule in the liver to form the liver cell plate and made up of a majority of the whole cells in the liver. In the specification, the "liver cells" are interchangeably used with "hepatic parenchymal cells".

In the present invention, "liver tissue" refers to a tissue constituted of a mass of liver lobules.

In the present invention, "angiogenesis" refers to a phenomenon where new blood vessels are formed from pre-existing vessels in, e.g., the liver and branched to construct a vascular network.

In the present invention, "liver fibrosis" refers to a phenomenon where the parenchymal tissue of a healthy liver is replaced by connective tissue due to repetitive damage and chronic hepatitis, and an extracellular matrix component such as collagen is accumulated.

In the present invention, the "cytokine production" refers to producing cytokines such as vascular endothelial growth factor (VEGF) and hepatocyte growth factor (HGF), which play a role in, e.g., angiogenesis, protection and repairment of cells. For example, in the liver to which the sheet-shaped cell culture of the present invention was applied, cytokines such as VEGF and HGF, which play a role in, e.g., angiogenesis, protection and repairment of cells, are produced.

In the present invention, the "reinforcing layer" refers to a layered material capable of reinforcing the structure without impairing the function of the sheet-shaped cell culture of the present invention.

The sheet-shaped cell culture preferably contains no scaffold (support). The scaffold is sometimes used in the technical field for attaching cells on the surface or inside thereof and maintaining the physical unity of a sheet-shaped cell culture. For example, a film formed of, e.g., polyvinylidene difluoride (PVDF), is known. However, the sheet-shaped cell culture of the present invention can maintain physical unity without such a scaffold. Furthermore, the sheet-shaped cell culture of the present invention preferably consists of substances derived from cells constituting the sheet-shaped cell culture, and does not contain any other substances.

The sheet-shaped cell culture of the present invention may contain optional cells in addition to skeletal myoblasts. The optional cells are not particularly limited as long as they form a sheet-shaped cell culture. For example, adhesion cells (adherent cells) are included. Examples of the adhesion cells include, adhesive somatic cells (for example, cardiomyocytes, fibroblasts, epithelial cells, endothelial cells, liver cells, pancreatic cells, kidney cells, adrenal gland cells, periodontal ligament cells, gingival cells, periosteal cells, skin cells, synovial cells, cartilage cells) and stem cells (for example, tissue stem cells such as myoblasts and cardiac stem cells, pluripotent stem cells such as embryonic stem cells and iPS (induced pluripotent stem) cells, and mesenchymal stem cells). The somatic cells may be differentiated from stem cells, particularly iPS cells (iPS cell-derived adherent cells). Non-limiting examples of the cells constituting a sheet-shaped cell culture include myoblasts, mesenchymal stem cells (for example, cells derived from bone marrow, adipose tissue, peripheral blood, skin, hair root, muscle tissue, endometrium, placenta and umbilical cord blood), cardiomyocytes, fibroblasts, cardiac stem cells, embryonic stem cells, iPS cells, synovial cells, cartilage cells, epithelial cells (for example, oral mucosal epithelial cells, retinal pigment epithelial cells and nasal mucosal epithelial cells), endothelial cells (for example, vascular endothelial cells), liver cells (for example, hepatic parenchymal cells), pancreatic cells (for example, pancreatic islet cells), kidney cells, adrenal gland cells, periodontal ligament cells, gingival cells, periosteal cells, and skin cells. Non-limiting examples of the iPS cell-derived adherent cells include iPS cell-derived cardiomyocytes, fibroblasts, epithelial cells, endothelial cells, liver cells, pancreatic cells, kidney cells, adrenal gland cells, periodontal ligament cells, gingival cells, periosteal cells, skin cells, synovial cells, and cartilage cells.

The cells constituting a sheet-shaped cell culture may be derived from an organism that can be treated with the sheet-shaped cell culture. Examples of the organism include, but are not limited to, a human, a non-human primate, a dog, a cat, a pig, a horse, a goat, sheep, a rodent (for example, a mouse, a rat, a hamster, a guinea pig) and a rabbit. Furthermore, the number of types of cells constituting a sheet-shaped cell culture is not particularly limited. A single type of cell or two types or more of cells may be used. In a case where the number of types of cells in the composition is two or more, the content ratio (purity) of the most common type of cell is 50% or more, preferably 60% or more, more preferably 70% or more, and further preferably 75% or more, at the time when completion of the formation of the composition of the present invention.

In an embodiment, the cells are autogenic cells. In a case where the sheet-shaped cell culture is used for implantation, "autogenic cells" herein refer to cells derived from a recipient.

In an embodiment, the cells are isogenic cells. Herein, the "isogenic cells" refer to cells derived from the same species as the recipient and similar species based on the genetical relatedness (in other words, no rejection reaction occurs after implantation to the recipient). For example, in a case where the recipient is a human, human cells of the similar species based on the genetical relatedness correspond to isogenic cells.

In an embodiment, the cells are allogeneic cells. Herein, the "allogeneic cells" refer to cells derived from an organism belonging to the same species as the recipient but different species based on the genetical relatedness (in other words, a rejection reaction occurs after implantation to the recipient). For example, in a case where the recipient is a human, human cells of the different species based on the genetical relatedness correspond to allogeneic cells.

In the specification, the "autogenic cells" are used interchangeably with "autogenic cells", and "isogenic cells" and "allogeneic cells" are interchangeably used with "xenogenic cells".

In an embodiment, cells are heterogenic cells. In a case where the sheet-shaped cell culture is used for implantation, the "heterogenic cells" herein refer to cells derived from an organism of different species from the recipient. For example, in a case where the recipient is a human, cells derived from a monkey or a pig correspond to the heterogenic cells.

The autogenic cells and isogenic cells are preferable in the present invention because no rejection reaction occurs even if they are implanted. However, it is possible to use allogeneic cells and heterogenic cells. In a case where the allogeneic cells and heterogenic cells are used, an immunosuppressive treatment is often required to suppress a rejection reaction.

The sheet-shaped cell culture can be produced by a commonly known method (see, for example, JP 2010-081829 A, JP 2011-110368 A). A method for producing a sheet-shaped cell culture includes a step of seeding cells on a culture substrate; a step of forming a sheet of the cells seeded; and a step of detaching the formed sheet-shaped cell culture from the culture substrate. However, the method is not limited to this. Before the step of seeding cells on a culture substrate, a step of freezing the cells and a step of thawing the cells may be performed. Further, after the step of thawing the cells, a step of washing the cells may be performed. These steps each can be performed by a commonly known process suitable for producing a sheet-shaped cell culture. In an embodiment, a step of proliferating the cells is not included after the step of thawing the cells and before the step of seeding the cells on a culture substrate.

The culture substrate is not particularly limited as long as a cell culture can be formed thereon. Examples of the culture substrate include containers made of various types of materials and solid or semi-solid surfaces in the containers. The structure and material of the container are preferably impermeable with a liquid such as a culture solution. Examples of the material include, but are not limited to, polyethylene, polypropylene, Teflon (registered trademark), polyethylene terephthalate, polymethyl methacrylate, nylon 6,6-polyvinyl alcohol, cellulose, silicon, polystyrene, glass, polyacrylamide, polydimethylacrylamide, and a metal (for example, iron, stainless, aluminum, copper, brass). Furthermore, it is also preferred that the container has at least one flat surface. Examples of such a container include, but are not limited to, a culture container having a bottom surface composed of a cell culture substrate capable of forming a cell culture and a liquid-impermeable side surface. Specific examples of the container include, but are not limited to, a cell culture plate and a cell culture bottle. The bottom surface of the container may be transparent or not. If the bottom surface of the container is transparent, it is possible to, e.g., observe cells and count cells through the underside of the container. Furthermore, the container may have a solid or semi-solid surface therein. Examples of the solid surface include plates and containers formed of various types of materials as mentioned above. Examples of the semi-solid surface include gel and soft polymer matrixes. The culture substrate may be prepared from any one of the above materials or a commercially available culture substrate may be used. A preferable culture substrate is not limited and, for example, a substrate having an adhesive surface suitable for forming a sheet-shaped cell culture. Specific examples thereof include a substrate having a hydrophilic surface, for example, a substrate whose surface is coated with a hydrophilic compound such as polystyrene treated with corona discharge, collagen gel or a hydrophilic polymer; and a substrate whose surface is coated with an extracellular matrix such as collagen, fibronectin, laminin, vitronectin, proteoglycan or glycosaminoglycan, or a cell-adhesion factor such as a cadherin family, a selectin family or an integrin family. Furthermore, such a substrate is commercially available (for example, Corning (registered trademark), TC-Treated Culture Dish, Corning). The culture substrate may be entirely or partially transparent or not.

The culture substrate may have a surface coated with a material whose physical properties change in response to stimulation such as temperature or light. Examples of the material that can be used include, but are not limited to, materials commonly known such as temperature responsive materials made of a homopolymer or copolymer of a (meth)acrylamide compound, a N-alkyl substituted (meth)acrylamide derivative (for example, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, or N-tetrahydrofurfuryl methacrylamide), a N,N-dialkyl substituted (meth)acrylamide derivative (for example, N,N-dimethyl (meth)acrylamide, N,N-ethyl methyl acrylamide, or N,N-diethyl acrylamide), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine), or a vinyl ether derivative (for example, methyl vinyl ether); and photoresponsive materials such as a light absorbing polymer having an azobenzene group, a copolymer of a vinyl derivative of triphenylmethane leucohydroxide and an acrylamide monomer, and a N-isopropylacrylamide gel containing spirobenzopyran (see, for example, JP H2-211865 A, JP 2003-33177 A). The physical properties, for example, hydrophilicity or hydrophobicity, of these materials are changed by applying a predetermined stimulation, with the result that detachment of a cell culture attached on the materials can be promoted. The culture plates coated with a temperature responsive material, which are commercially available (for example, UpCell (registered trademark) of CellSeed Inc.), can be used in a method for producing a sheet-shaped cell culture.

Although the culture substrate may have any shape, the shape is preferably flat. Furthermore, the area of the culture substrate is not particularly limited but may be, for example, about 1 cm² to about 200 cm², about 2 cm² to about 100 cm², or about 3 cm² to about 50 cm². Examples of a culture substrate include a circular culture plate having a diameter of 10 cm. In this case, the area is 56.7 cm².

The culture substrate may be coated (covered or coating) with a serum. Use of the culture substrate coated with a serum makes it possible to form a higher-density sheet-shaped cell culture. "Coated with serum" refers to a state where serum components are attached to the surface of a culture substrate. Such a state can be obtained by treating a culture substrate with a serum but is not limited. The treatment with a serum includes bringing the serum in contact with a culture substrate, and, if necessary, incubating for a predetermined period of time.

The serum for coating a culture substrate is commercially available or can be prepared from the blood collected from a desired organism in accordance with a routine method. As a specific method, for example, collected blood is allowed to stand still at room temperature for about 20 minutes to about 60 minutes to coagulate, and the coagulate is centrifuged at about 1000 × g to about 1200 × g to collect the supernatant.

In the case of incubation on a culture substrate, the serum undiluted or diluted may be used. For dilution, any medium may be used. Examples of the medium include, but are not limited to, water, physiological saline, various types of buffer solutions (for example, PBS, HBSS), various types of liquid mediums (for example, DMEM, MEM, F12, DMEM/F12, DME, RPMI1640, MCDB (MCDB102, 104, 107, 120, 131, 153, 199, etc.) L15, SkBM, and RITC80-7). The dilute concentration is not particularly limited as long as the serum components can adhere onto a culture substrate. The dilute concentration is, for example, about 0.5% to about 100% (v/v), preferably about 1% to about 60% (v/v), and more preferably about 5% to about 40% (v/v).

The incubation time is also not particularly limited as long as the serum components can adhere onto a culture substrate. The incubation time is, for example, about 1 hour to about 72 hours, preferably about 2 hours to about 48 hours, more preferably about 2 hours to about 24 hours, and further preferably about 2 hours to about 12 hours. The incubation temperature is also not particularly limited as long as the serum components can adhere onto a culture substrate. The incubation temperature is, for example, about 0°C to about 60°C, preferably about 4°C to about 45°C, and more preferably room temperature to about 40°C.

After completion of incubation, the serum may be discarded. As a method of discarding the serum, a routine method for discarding a liquid such as suction with a pipette and decantation can be used. In a preferred embodiment of the present invention, after the serum is discarded, the culture substrate may be washed with a serum-free washing solution. The serum-free washing solution is not particularly limited as long as it is a liquid medium containing no serum and having no negative effect on the serum components attached to a culture substrate. The examples of the serum-free washing solution include, but are not limited to, water, physiological saline, various types of buffer solutions (for example, PBS, HBSS), various types of liquid mediums (for example, DMEM, MEM, F12, DMEM/F12, DME, RPMI1640, MCDB (MCDB102, 104, 107, 120, 131, 153, 199) L15, SkBM and RITC80-7). As the washing method, a routine culture-substrate washing method can be used. Examples of the washing method include, but are not limited to, a method of adding a serum-free washing solution onto a culture substrate, stirring it for a predetermined period (for example, about 5 seconds to about 60 seconds), followed by discarding it.

In the present invention, a culture substrate may be coated with a growth factor. Herein the "growth factor" refers to a substance that promotes cell proliferation compared to a case in the absence of the substance. Examples of the growth factor include epidermal growth factor (EGF), vascular endothelial growth factor (VEGF) and fibroblast growth factor (FGF). The method for coating a culture substrate with a growth factor, a discarding method and a washing method are basically the same as those for a serum except that dilution concentration during incubation is, for example, about 0.0001 µg/mL to about 1 µg/mL, preferably about 0.0005 µg/mL to about 0.05 µg/mL, and more preferably about 0.001 µg/mL to about 0.01 µg/mL.

In the present invention, a culture substrate may be coated with a steroid. Herein, the "steroid" refers to a compound having a steroid nucleus and having a negative effect such as adrenocortical insufficiency and Cushing syndrome on a living body. Examples of the compound include, but are not limited to, cortisol, prednisolone, triamcinolone, dexamethasone and betamethasone. The method for coating a culture substrate with a steroid, a discarding method and a washing method are basically the same as those for a serum except that dilution concentration during incubation is, for example, about 0.1 µg/mL to about 100 µg/mL, preferably about 0.4 µg/mL to about 40 µg/mL, and more preferably about 1 µg/mL to about 10 µg/mL.

A culture substrate may be coated with any one of a serum, a growth factor and a steroid, or with any combination of them, more specifically, a combination of a serum and a growth factor, a serum and a steroid, a serum, a growth factor and a steroid, or a growth factor and a steroid. In the case of coating with a plurality of components, these components may be mixed and simultaneously applied for coating or applied in separate steps.

Immediately after the culture substrate is coated with e.g., a serum, cells may be seeded. Alternatively, the coated culture substrate is stored, and thereafter, cells can be seeded. The coated substrate can be stored for a long time by keeping it, for example, at about 4°C or less, preferably about -20°C or less, and more preferably about - 80°C or less.

Cells can be seeded to a culture substrate by a routine method in the conditions commonly known. Cells can be seeded to a culture substrate, for example, by pouring a cell suspension having cells suspended in a culture solution in a culture substrate (culture container). The cell suspension can be poured by a tool suitable for pouring operation such as a dropper or a pipette.

Cells can be seeded at a density of, e.g., about 7.1 × 10⁵ cells/cm² to about 3.0 × 10⁶ cells/cm², about 7.3 × 10⁵ cells/cm² to about 2.8 × 10⁶ cells/cm², about 7.5 × 10⁵ cells/cm² to about 2.5 × 10⁶ cells/cm², about 7.8 × 10⁵ cells/cm² to about 2.3 × 10⁶ cells/cm², about 8.0 × 10⁵ cells/cm² to about 2.0 × 10⁶ cells/cm², about 8.5 × 10⁵ cells/cm² to about 1.8 × 10⁶ cells/cm², about 9.0 × 10⁵ cells/cm² to about 1.6 × 10⁶ cells/cm², or about 1.0 × 10⁶ cells/cm² to about 1.6 × 10⁶ cells/cm².

In a case where skeletal myoblasts are prepared from the striated muscle tissue, fibroblasts are contained in the cell population thus prepared. In a case where the sheet-shaped cell culture of the present invention is produced, if a cell population containing skeletal myoblasts prepared from the striated muscle tissue is used, a predetermined amount of fibroblasts is inevitably contained in the cell population. Fibroblasts are well known in the technical field and can be identified by a marker such as TE-7 (see, for example, Rosendaal et al., J Cell Sci. 1994; 107 (Pt 1): 29-37, Goodpaster et al., J Histochem Cytochem. 2008; 56 (4): 347-58).

In an embodiment, cells forming the sheet-shaped cell culture of the present invention include skeletal myoblasts prepared from striated muscle tissue. Accordingly, the cell population to be used in producing the sheet-shaped cell culture of the present invention may contain skeletal myoblasts and fibroblasts. In an embodiment, the cell population to be used in producing the sheet-shaped cell culture of the present invention has a CD56 positive rate of 50% or more, 60% or more, 65% or more, 70% or more, 75% or more, 80% or more, 85% or more or 90% or more, and preferably 60% or more.

The cell population used in the production of the sheet-shaped cell culture of the present invention may contain fibroblasts, but when the content of fibroblasts is too high, the content of skeletal myoblasts decreases, which is not preferable. Accordingly, in an embodiment, the cell population to be used in producing the sheet-shaped cell culture of the present invention may have a TE7 positive rate of 50% or less, 40% or less, 35% or less, 30% or less, 25% or less, 20% or less, 150 or less or 10% or less, and preferably 40% or less.

The cell population to be used in producing the sheet-shaped cell culture of the present invention may contain cells other than skeletal myoblasts and fibroblasts but the content of the other cells is preferably as low as possible. Accordingly, the higher the total value of the CD56 positive rate and the TE7 positive rate, the more preferable. The total value is, for example, 80% or more, 85% or more, 90% or more, 95% or more, 96% or more, 97% or more, 98% or more or 99% or more and preferably 90% or more.

The thickness of the sheet-shaped cell culture of the present invention is not particularly limited. In a case where a single-layer sheet is used as the sheet-shaped cell culture, the thickness of the single-layer sheet is, usually a thickness of a single cell or more. The thickness varies depending on the types of cells constituting the sheet-shaped cell culture. The sheet-shaped cell culture of the present invention has a thickness of 30 um or more in an embodiment and 50 um or more in a preferred embodiment. The thickness of the sheet-shaped cell culture of the present invention falls within the range of, for example, 30 um to 200 um, preferably 50 um to 150 µm, and more preferably 60 um to 100 um In a case where a laminate sheet is used as the sheet-shaped cell culture, the thickness of the laminate sheet is defined to fall within a value given by the thickness of the single layer sheet × the number of lamination layers. Accordingly, in an embodiment where a laminate sheet composed of 5 single-layer sheets is used, the thickness thereof is 150 um or more, and preferably 250 um or more ad falls within the range of, for example, 150 um to 1000 um, preferably 250 um to 750 µm, and more preferably 300 um to 500 um.

Accordingly, the thickness of the sheet-shaped cell culture of the present invention is, for example, 30 um to 1000 um, preferably 50 um to 750 um, 50 um to 500 um or 60 um to 500 µm.

In an embodiment, the sheet-shaped cell culture of the present invention is extremely fragile. It is sometimes difficult to handle it. Accordingly, the sheet-shaped cell culture of the present invention may further have a reinforcement layer for the purpose of making handling easier and reducing a risk of breakage. The reinforcement layer is not limited as long as it can reinforce the structure of the sheet-shaped cell culture of the present invention without impairing the function thereof. The reinforcement layer may contain, for example, a gel and/or a polymer. However, since the sheet-shaped cell culture of the present invention is to be implanted to a living body, the reinforcement layer is preferably a biocompatible reinforcement layer containing, e.g., a biocompatible gel or polymer.

The gel to be used in the reinforcement layer, preferably the biocompatible gel, is not limited as long as it does not adversely affect a living body when introduced. Examples of the gel include, but are not limited to, fibrin gel, fibrinogen gel, gelatin gel and collagen gel.

The polymer to be used in the reinforcement layer, preferably the biocompatible polymer, is not limited as long as it does not adversely affect a living body when introduced. Examples of the polymer include, but are not limited to, polylactic acid, polydioxano, polyglycapro and collagen.

As the method for forming a reinforcement layer containing a biocompatible gel, a method commonly known in the technical field can be used. Examples of the method include, but are not limited to, a method of spraying a biocompatible gel or a polymer or components thereof on a sheet-shaped cell culture; a method of laminating a sol-like biocompatibility substance on a sheet-shaped cell culture and converting the substance into a gel; a method of soaking a sheet-shaped cell culture in a liquid-state gel and solidifying the gel; and other methods, for example, a method disclosed in JP 2016-52271A.

Since the reinforcement layer is used for making handling of the sheet-shaped cell culture of the present invention easier and reducing a risk of breakage, the reinforcement layer preferably has a strength of a predetermined level or more and, in addition, elasticity. As the unit for evaluating strength for a construct containing a gel or a polymer, for example, jelly strength is commonly known. As the unit for evaluating strength for a sheet-form construct, for example, a tensile fracture load is known. A method for measuring jelly strength is disclosed, for example, in JIS K 6503. The tensile fracture load refers to a maximum value of the load, which is measured by pulling both ends of, e.g., a sheet-shaped cell culture, in the horizontal direction until it breaks. The measurement method thereof is disclosed, for example, in JP 2016-52272 A.

The tensile fracture load of the reinforcement layer of the sheet-shaped cell culture of the present invention is not limited, but it may be about 0.010 N or more, about 0.015 N or more, about 0.020 N or more, about 0.025 N or more, about 0.030 N or more, about 0.035 N or more, about 0.040 N or more, or about 0.045 N or more, or may fall within the range of about 0.010 N to about 0.200 N, about 0.015N to about 0.100 N, or about 0.020 N to about 0.50 N. The strength of the sheet-shaped cell culture having a reinforcement layer may be about 1.5 times or more, about 2 times or more, about 3 times or more, about 4 times or more, about 5 times or more, about 6 times or more, about 7 times or more, about 8 times or more, about 9 times or more, about 10 times or more, and furthermore may fall in the range of about 1.5 times to about 20 times, about 2 times to about 15 times, about 2.5 times to about 10 times as large as the strength of the sheet-shaped cell culture having no reinforcement layer.

In a case where a sheet-shaped cell culture having a reinforcement layer is applied to an application site, it is preferable that the sheet is applied such that the reinforcement layer is not directly in contact with the site. In other words, the sheet-shaped cell culture is preferably applied such that the sheet-shaped cell culture is positioned between an application site and the reinforcement layer.

In an embodiment, in a case where the sheet-shaped cell culture of the present invention is applied to a tissue, the sheet may be applied in combination with a composition and/or graft, etc., for promoting healing. Examples of the composition and/or graft for promoting healing include, but are not limited to, a graft containing a vascular pedicle such as an omentum majus piece, fibrin gel and Adspray (registered trademark). In a preferred embodiment, the sheet-shaped cell culture of the present invention is applied in combination with a graft containing a vascular pedicle. A representative example of the graft containing a vascular pedicle is an omentum majus piece.

The composition and/or graft for promoting healing may be a discrete composition or graft from the sheet-shaped cell culture of the present invention, or may be integrated into, for example, a sheet-shaped cell culture or reinforcement layer.

In a case where the sheet-shaped cell culture of the present invention is applied together with a discrete composition and/or graft for promoting heating, the composition and/or graft may be applied before or after the sheet-shaped cell culture is applied. In a case where the composition and/or graft is applied before the sheet-shaped cell culture is applied, the composition and/or graft is applied so as to be positioned between an application site and the sheet-shaped cell culture. More specifically, the composition and/or graft is first applied to an application site, and thereafter, the sheet-shaped cell culture (optionally containing a reinforcement layer) is applied over the composition and/or graft. In a case where the composition and/or graft is applied after the sheet-shaped cell culture is applied, the composition and/or graft is applied to an application site with the sheet-shaped cell culture (optionally containing a reinforcement layer) interposed between them. More specifically, the sheet-shaped cell culture is first applied to an application site, and then, the composition and/or graft is applied on the sheet-shaped cell culture.

A mechanism for tissue regeneration is considered, for example, due to a paracrine effect, which is bought by sustained-release of the composition of the present invention of e.g., cytokines such as VEGF, HGF and collagen, which play a role in, e.g., angiogenesis, and protection and repair of cells at an affected site; and/or, due to an autocrine effect, which is brought by e.g., collagen production promoted by activation of progenitor cells or stem cells of the tissue around the application site.

The size of the sheet-shaped cell culture of the present invention is not particularly limited as long as it can cover a predetermined part of living tissue. If the sheet-shaped cell culture is, for example, circular, the diameter thereof is 10 to 55 mm, 15 to 50 mm, 20 to 45 mm, 25 to 40 mm, or 30 to 35 mm.

### [Method for Producing the Sheet-shaped cell culture of the Present Invention]

Another aspect of the present invention relates to a method for producing the sheet-shaped cell culture of the present invention, including a step of seeding a cell population containing skeletal myoblasts on a culture substrate; a step of forming a sheet of the cell population seeded to form a sheet-shaped cell culture; and detaching the formed sheet-shaped cell culture from the culture substrate (sometimes referred to as "the production method of the present invention").

### [Method for Treating Liver Dysfunction]

Another aspect of the present invention relates to a method for treating liver dysfunction, including a step of applying the sheet-shaped cell culture of the present invention to a site exhibiting liver dysfunction (sometimes referred to as "the treatment method of the present invention").

In the present invention, "site exhibiting liver dysfunction" refers to a liver site at which liver function is impaired or suppressed and/or a liver site that lost liver function. In an embodiment, the site exhibiting liver dysfunction is a site of the liver surface at which liver function is impaired or suppressed and/or a site of the liver surface losing liver function. In an embodiment, the site exhibiting liver dysfunction is a site of the liver surface exhibiting hepatitis, liver fibrosis, liver cirrhosis, liver cancer, or liver failure. In an embodiment, the site exhibiting liver dysfunction is a site of the liver surface exhibiting compensated cirrhosis or decompensated cirrhosis. In an embodiment, the site exhibiting liver dysfunction is a site of the liver surface exhibiting initial- or terminal-stage decompensated cirrhosis In an embodiment, the site exhibiting liver dysfunction is a site of the surface of residual liver exhibiting liver failure after liver cancer resection.

In an embodiment, in the treatment method of the present invention, the step of applying the sheet-shaped cell culture of the present invention to a site exhibiting liver dysfunction is a step of attaching the sheet-shaped cell culture of the present invention to a site exhibiting liver dysfunction.

In an embodiment, in the treatment method of the present invention, the step of applying the sheet-shaped cell culture of the present invention to a site exhibiting liver dysfunction is a step of implanting the sheet-shaped cell culture of the present invention to a site exhibiting liver dysfunction.

In an embodiment, in the treatment method of the present invention, the step of applying the sheet-shaped cell culture of the present invention to a site exhibiting liver dysfunction is a step of engrafting the sheet-shaped cell culture of the present invention in a site exhibiting liver dysfunction.

In an embodiment, in the treatment method of the present invention, the step of applying the sheet-shaped cell culture of the present invention to a site exhibiting liver dysfunction is a step of implanting the sheet-shaped cell culture of the present invention into the liver surface by an implantation device. In an embodiment, the implantation device is a medical device that can apply the sheet-shaped cell culture of the present invention to the liver surface. The implantation device is, for example, a medical device having a planar structure capable of supporting and detaching a sheet-shaped cell culture. In view of operating it within the abdominal cavity, the implantation device preferably has a form that can be inserted and passed through a cylindrical body which has been inserted in a body cavity in an endoscopic surgery.

### [Composition and Others Containing Sheet-shaped cell culture of the Present Invention]

Another aspect of the present invention relates to, e.g., a composition (for example, a pharmaceutical composition), graft and medical product containing the sheet-shaped cell culture of the present invention (sometimes referred to as "the composition and others" of the present invention).

The composition and others of the present invention can contain not only the sheet-shaped cell culture of the present invention but also various additional components. Examples of the additional components include a pharmaceutically acceptable carrier, a component enhancing viability, engraftment and/or functionality of a sheet-shaped cell culture and components useful for, e.g., regenerating and/or healing a living tissue or promotion thereof, and/or a graft. Those skilled in the art familiar with the additional components and can appropriately select an additional component commonly known and use it. Furthermore, in the composition and others of the present invention, an additional component may be used in combination with the sheet-shaped cell culture of the present invention.

In an embodiment, the composition and others of the present invention are provided for treating liver dysfunction or improving liver function.

In an embodiment, the composition and others of the present invention are provided for application to the liver after liver cancer resection.

In an embodiment, the composition and others of the present invention are provided for promoting proliferation of the liver cells, regeneration of the liver tissue and/or angiogenesis.

In an embodiment, the composition and others of the present invention are provided for suppressing liver fibrosis.

In an embodiment, the composition and others of the present invention are provided for treating liver dysfunction through cytokine production.

In an embodiment, the composition and others of the present invention are provided for treating compensated cirrhosis or decompensated cirrhosis. In an embodiment, the composition and others of the present invention are provided for treating initial- or terminal-stage decompensated cirrhosis. In an embodiment, the composition and others of the present invention are provided for treating the residual liver exhibiting liver failure after liver cancer resection.

### [Use in Producing Medicine for Treating Liver Dysfunction]

Another aspect of the present invention relates to use of the sheet-shaped cell culture of the present invention or the composition and others of the present invention (sometimes referred to as "use in producing the medicine of the invention") in producing a medicine for treating liver dysfunction.

### [Use for Treating Liver Dysfunction]

Another aspect of the present invention relates to use of the sheet-shaped cell culture of the present invention, or the composition and others of the present invention (sometimes referred to as "use of the present invention") for treating liver dysfunction.

The present invention will be more specifically described with reference to the following Examples, which show specific examples of the present invention and would not limit the present invention.

### Examples

### Example 1. Preparation of Sheet-shaped cell culture

### (1) Preparation of Cells

The striated muscular tissue of a lower limb of each mouse (C57BL/6J, CLEA Japan, Inc.) was collected under general anesthesia. The tissue collected was treated with an enzymatic digestive fluid containing collagenase and trypsin to separate into single cells. The single cells were cultured in MCDB131 medium containing 20% FBS at 37°C and in a 5% CO₂ condition until the cells reached confluence.

### (2) Preparation of Sheet-shaped cell culture

On the day when a sheet-shaped cell culture was implanted to partial-hepatectomy model mice shown Examples 2 and 3 and liver-fibrosis model mice shown in Example 4, the cells cultured in the above (1) were collected, and then, the cells (3.0 × 10⁶) were seeded in a 24-well temperature-responsive culture plate (UpCell (registered trademark), CellSeed Inc.) and cultured in a 20% FBS-containing DMEM/F12 medium for 12 hours. Thereafter, the temperature was decreased up to 20°C and a sheet-shaped cell culture was collected by detaching it from the temperature-responsive culture plate.

Hereinafter, liver dysfunction model mice (70% partial-hepatectomy model mice, 90% partial-hepatectomy model mice, and liver-fibrosis model mice) were prepared, and then, the sheet-shaped cell culture was implanted to the surface of mouse livers. The therapeutic effect of a sheet-shaped cell culture on liver dysfunction was investigated.

### Example 2. Treatment of 70% Partial-Hepatectomy Model Mice with Sheet-shaped cell culture

### (1) Preparation of partial-hepatectomy model mice

The mice used in Example 1 were divided into a sheet-treated group (n = 5) and a control group (n = 5) in an appropriate time. After the body hair of the abdomen of each of the mice was shaved under general anesthesia, the mice were fixed in the supine X-shaped position on a heating pad of 37°C with tape. The skin of the abdomen was disinfected with 70% ethanol Midline laparotomy was performed by cutting the abdominal skin and muscle in a length of 3 cm to expose the abdominal organs and liver. 70% of the liver was resected by resection of the middle and lateral lobes of the liver, leaving the right lobe, caudate lobe, and inferior vena cava. Immediately after partial hepatectomy, in the sheet-treated group, the sheet-shaped cell culture obtained in Example 1 was implanted to the surface of the residual liver and sutured with a suture thread (PDS PLUS (registered trademark), Johnson & Johnson) only at a single point (Fig. 1) In contrast, immediately after the partial hepatectomy, in the control group, suturation was performed with a suture thread to the residual liver in the absence of the sheet-shaped cell culture obtained in Example 1 only at a single point of the site corresponding to the implantation site of the sheet-shaped cell culture in the sheet-treated group. The blood vessels and bile duct were ligated; the abdominal cavity and the organs were washed with physiological saline; and the abdomen was closed with a suture thread. Thereafter, in the sheet-treated group and control group, the skin of the abdomen around the suture thread was disinfected with 70% ethanol. Thereafter, in order to make up for fluid loss due to e.g., bleeding during the surgery, an aseptic isotonic solution (0.5 mL) was subcutaneously injected.

### (2) Histological Examination of Liver

### (2-1) Preparation of Liver Section

Two and three days after implantation of the sheet-shaped cell culture, the abdomen was opened again under general anesthesia in the sheet-treated group and control group and the site having the sheet-shaped cell culture (a site where a sheet-shaped cell culture is confirmed by the naked eye) implanted in the sheet-treated group and the corresponding site in the control group were taken out. The sites taken out were soaked in ethanol for 3 hours, subsequently in xylene for 3 hours, and further in melted paraffin, overnight. After solidification by cooling paraffin, sections were prepared using a microtome. The sections were dried and deparaffinized.

### (2-2) Staining with Hematoxylin and Eosin (HE)

The sections (two days after implantation of the sheet-shaped cell culture) obtained in the above (2-1) were stained with a hematoxylin solution (Mayer's hematoxylin, MUTO PURE CHEMICALS CO., LTD.) for 10 minutes. Subsequently, the sections were washed with running water for 15 minutes and stained with an eosin solution (1% eosin Y solution, manufactured by MUTO PURE CHEMICALS CO., LTD.) for 10 minutes. Thereafter, the sections were dewatered with alcohol and naturally dried at room temperature. The sections stained were observed by an optical microscope at 200-fold magnification.

As a result, it was confirmed that the sheet-shaped cell culture remained in close contact with the surface of the liver, at the implantation site, two days after the sheet-shaped cell culture was implanted (Fig. 2). From this, it was found that the sheet-shaped cell culture implanted is engrafted in the liver surface.

### (2-3) Immunofluorescence staining

The sections (2 days after implantation of the sheet-shaped cell culture) obtained in the above (2-1) were washed with phosphate-buffered physiological saline (PBS), and subsequently, PBS was removed. The sections were soaked in a 0.5% (v/v) Triton (registered trademark) X-100 solution (penetration treatment solution) dissolved in PBS and incubated for 15 minutes at room temperature. The penetration treatment solution was removed, the sections were washed with PBS, and subsequently, PBS was removed. The sections were soaked in a 3% bovine serum albumin solution (blocking solution) dissolved in PBS and incubated for 60 minutes at room temperature. The blocking solution was removed, the sections were washed with PBS, and subsequently, PBS was removed The sections were soaked in a primary-antibody solution (containing anti-desmin antibody (ab8470, Abeam)) and incubated for one hour at room temperature. The primary antibody solution was removed, the sections were washed with PBS, and subsequently, PBS was removed. The sections were soaked in a secondary antibody solution (containing a fluorescently labeled goat anti-mice IgG antibody (A-11001, Thermo Fisher Scientific)) and incubated for 30 minutes at room temperature. The secondary antibody solution was removed, the sections were washed with PBS, and subsequently, PBS was removed. The sections were soaked in Hoechst solution (SD024, Dojindo Laboratories) and incubated for 30 minutes at room temperature. The Hoechst solution was removed, the sections were washed with PBS, and subsequently, PBS was removed. The sections stained were observed by a fluorescence microscope at 200-fold magnification.

As a result, it was confirmed that the sheet-shaped cell culture remained in close contact with the surface of the liver at the implantation site, two days after implantation of the sheet-shaped cell culture (Fig. 3). From this, it was found that the sheet-shaped cell culture implanted is engrafted in the liver surface.

### (2-4) Immunostaining

The sections (2 and 3 days after implantation of the sheet-shaped cell culture) obtained the above (2-1) were washed with phosphate-buffered physiological saline (PBS), and subsequently, PBS was removed. The sections were soaked in a 0.5% (v/v) Triton (registered trademark) X-100 solution (penetration treatment solution) dissolved in PBS and incubated for 15 minutes at room temperature. The penetration treatment solution was removed, the sections were washed with PBS, and subsequently, PBS was removed. The sections were soaked in a 3% bovine serum albumin solution (blocking solution) dissolved in PBS and incubated for 60 minutes at room temperature. The blocking solution was removed, the sections were washed with PBS, and subsequently, PBS was removed The sections were soaked in a primary antibody solution (containing anti-Ki67 antibody (ab16667, Abeam)) and incubated for one hour at room temperature. The primary antibody solution was removed, the sections were washed with PBS, and subsequently, PBS was removed. The sections were soaked in a secondary-antibody solution (containing an enzyme-labeled antibody (LSAB2 System-HRP (K0672), Agilent Technologies)) and incubated for 30 minutes at room temperature The secondary antibody solution was removed, the sections were washed with PBS, and subsequently, PBS was removed. The sections were soaked in an enzyme-substrate solution (containing an enzyme substrate (LSAB2 System-HRP (K0672), Agilent Technologies)) and incubated for 30 minutes at room temperature to produce a color reaction. The enzyme-substrate solution was removed, the sections were washed with PBS, and subsequently, PBS was removed. The sections stained were observed by an optical microscope.

As a result, it was confirmed, in each of the sheet-treated group and control group, that Ki67 (cell proliferation marker)-positive cells are present at the implantation sites 2 and 3 days after implantation of the sheet-shaped cell culture (Fig. 4). Particularly, at the implantation site 2 days after implantation of the sheet-shaped cell culture, the number of Ki67-positive cells was higher in the sheet-treated group than in the control group. Thus, it was found that the proliferation of liver cells and regeneration of the liver tissue are promoted by implantation of the sheet-shaped cell culture to the liver surface.

### (2-5) Calculation of Liver-Weight/Body-Weight Ratio

After 1, 2, 3, and 7 days from implantation of the sheet-shaped cell culture, the body weight of mice in the sheet-treated group and control group was measured, at the same time, the abdominal cavity was opened again under general anesthesia and the weight of the liver (liver weight) of mice was measured. On the basis of the body weights and liver weights measured, the liver-weight/bodyweight ratios at 1, 2, 3, and 7 days after implantation of the sheet-shaped cell culture were individually calculated.

As a result, it was confirmed, in each of the sheet-treated group and control group, that the liver-weight/body-weight ratio increases as the days pass after surgery (Fig. 5). In the sheet-treated group compared to the control group, there was a tendency that the liver-weight/body-weight ratio is larger. Thus, it was found that the proliferation of liver cells and regeneration of the liver tissue are promoted by implantation of the sheet-shaped cell culture to the liver surface.

### (3) Clinical Chemistry Test for the Liver

### (3-1) Preparation of Lysate

After 1, 2, 3, and 7 days from implantation of the sheet-shaped cell culture, the abdomen of mice was opened again under general anesthesia in the sheet-treated group and control group, at the same time, the site having the sheet-shaped cell culture (a site where the sheet-shaped cell culture is confirmed by the naked eye) implanted in the sheet-treated group and the corresponding site in the control group were taken out. The sites taken out were washed with cooled PBS and cut into small pieces while they were cooled on the ice. These pieces were put in a homogenizer and RIPA buffer (containing a protease inhibitor) was added thereto. The mixture was homogenized, ultrasonically treated, and then centrifuged at 10,000 × g and 4°C for 20 minutes to obtain the supernatant (lysate).

### (3-2) Western Blotting

To the lysates (2 and 3 days after implantation of the sheet-shaped cell culture) obtained in the above (3-1), a 2 × SDS sample buffer (125 mM Tris-HCl, 4% SDS, 20% glycerol, 0.01% bromophenol blue, and 10% 2-mercaptoethanol) was added so as to obtain a final concentration of 1X. The mixture solution was stirred. The mixture solution was heated at 95°C for 5 minutes and subjected to polyacrylamide gel electrophoresis. Subsequently, the gel electrophoresed was transferred to PVDF membrane. The membrane was blocked with a 2% skim milk solution, and thereafter, reacted with a peroxidase-labeled anti-Akt antibody (9272, Cell Signaling Technology), an anti-p-Akt (Ser473) antibody (9271, Cell Signaling Technology), an anti-CD31 antibody (ab124432, Abcam), and an anti-β-actin antibody (A2066, Sigma Aldrich). Subsequently, a horseradish peroxidase (HRP)-labeled secondary antibody (NA934V, Cytiva) was reacted, and then, a peroxidase substrate solution (RPN2236, Cytiva) was added to emit light. Imaging was carried out using a chemiluminescence imaging device.

As a result, it was confirmed that 2 days after implantation of the sheet-shaped cell culture, the expression levels of Akt (cell proliferation marker) and p-Akt (Ser473) (cell proliferation marker) in the sheet-treated group are higher than those of the control group (Fig. 6). Thus, it was found that the proliferation of liver cells and regeneration of the liver tissue are promoted by implantation of the sheet-shaped cell culture to the liver surface.

Furthermore, it was found that 2 and 3 days after implantation of the sheet-shaped cell culture, the expression level of CD31 (angiogenesis marker) in the sheet-treated group is higher than that of the control group (Fig. 7). Thus, it was found that angiogenesis is promoted by implantation of the sheet-shaped cell culture to the liver surface.

### (3-3) Real-time PCR

Total RNA was purified from lysates (lysates on 1, 2, 3, and 7 days after implantation of the sheet-shaped cell culture) obtained in the above (3-1) using a commercially available RNA purification kit (Rneasy Mini Kit, QIAGEN). Thereafter, the RNA samples were subjected to RNA reverse transcription and real-time PCR by use of a commercially available real-time PCR kit (Reverse Transcription System, Promega) to obtain the expression levels of mRNA of VEGF and HGF contained in the lysate obtained in the above (3-1). For VEGF, the forward primer was represented by 5'-GAAGGAGAGCAGAAGTCCCA-3' (SEQ ID NO: 1) and the reverse primer was represented by 5'-ACACAGGACGGCTTGAAGAT-3' (SEQ ID NO: 2). Furthermore, for HGF, the forward primer was represented by 5'-TGACCTGCAATGGTGAAAGC-3' (SEQ ID NO: 3) and the reverse primer represented by 5'-GGGTCAAGAGTGTAGCACCA-3' (SEQ ID NO: 4). The thermal cycler settings were as shown in Table 1.

**Table 1. Thermal cycler settings**

| [Table 1] | | |
|---|---|---|
| Temperature | Time | Number of cycles |
| 98°C | 1 minute | - |
| 98°C | 10 seconds | 30 times |
| 55°C | 15 seconds | |
| 72°C | 40 seconds | |
| 72°C | 2 minutes | - |

As a result, it was confirmed with regard to VEGF (angiogenesis marker) that the expression level of mRNA tends to increase as the days pass after surgery in both of the sheet-treated group and control group. (Fig. 8). In contrast, with regard to HGF (angiogenesis marker), there was no tendency that the expression level of mRNA increases as the days pass after surgery in both of the sheet-treated group and control group. Thus, it was found that the angiogenesis in the liver is promoted by implantation of the sheet-shaped cell culture to the liver surface.

### (3-4) ELISA

The lysates (one day and three days after implantation of the sheet-shaped cell culture) obtained in the above (3-1) were subjected to ELISA using a commercially available ELISA kit (ab223862, Abcam) to obtain the expression levels of HGF contained in the lysates obtained in the above (3-1) .

As a result, one day after implantation of the sheet-shaped cell culture, it was confirmed in the sheet-treated group that the expression level of HGF (angiogenesis marker) is higher than that of the control group (Fig. 9). In contrast, 3 days after implantation of the sheet-shaped cell culture, in the sheet-treated group, the expression level of HGF (angiogenesis marker) is lower than that of the control group. Thus, it was found that HGF expression is increased in an early stage by implantation of the sheet-shaped cell culture to the liver surface.

### Example 3. Treatment of 90% Partial-Hepatectomy Model Mice with Sheet-shaped cell culture

### (1) Preparation of partial-hepatectomy model mice

The mice used in Example 1 were divided into a sheet-treated group (n = 7) and a control group (n = 12). After the body hair of the abdomen of each of the mice was shaved under general anesthesia, the mice were fixed in the supine X-shaped position on a heating pad of 37°C with tape. The skin of the abdomen was disinfected with 70% ethanol Midline laparotomy was performed by cutting the abdominal skin and muscle in a length of 3 cm to expose the abdominal organs and liver. The middle lobe and lateral lobe of the liver were excised, thereby removing 90% of the liver and the right lobe, caudate lobe and inferior vena cava were allowed to remain. Immediately after the partial hepatectomy, in the sheet-treated group, the sheet-shaped cell culture obtained in Example 1 was implanted to the surface of the residual liver and sutured with a suture thread (PDS PLUS (registered trademark), Johnson & Johnson) only at a single point. In contrast, immediately after the partial hepatectomy, in the control group, suturation was performed with a suture thread to the residual liver in the absence of the sheet-shaped cell culture obtained in Example 1 only at a single point of the site corresponding to the implantation site of the sheet-shaped cell culture in the sheet-treated group. The blood vessels and bile duct were ligated; the abdominal cavity and the organs were washed with physiological saline; and the abdomen was closed with a suture thread. Thereafter, in the sheet-treated group and control group, the skin of the abdomen around the suture thread was disinfected with 70% ethanol. Thereafter, in order to make up for fluid loss due to e.g., bleeding during the surgery, an aseptic isotonic solution (0.5 mL) was subcutaneously injected.

### (2) Calculation of Survival Rate after Partial Hepatectomy

The survival rates of mice in the sheet-treated group (n = 7) and control group (n = 12) were calculated 6, 12, 24, 36, and 47 hours after implantation of the sheet-shaped cell culture. Each of the survival rates was calculated by dividing the number of survival mice at each of the time points by the total number of mice of each group.

As a result, all mice died within 25 hours after surgery in the control group but, in the sheet-treated group, it was confirmed that some mice are still alive even beyond 35 hours after surgery (Fig. 10). Thus, it was found that the survival rate of mice is improved by implantation of the sheet-shaped cell culture to the liver surface.

### Example 4. Treatment of Liver-Fibrosis Model Mice with Sheet-shaped cell culture

### (1) Preparation of Liver-Fibrosis Model Mice

The mice used in Example 1 were divided into a sheet-treated group (n = 5) and a control group (n = 5). To mice of individual groups, 150 mg/kg thioacetamide (TAA) (204-00881, FUJIFILM Wako Pure Chemical Corporation) was intraperitoneally injected twice a week (Fig. 11A). Subsequently, the body hair of the abdomen of each of the mice was shaved under general anesthesia, and the mice were fixed in the supine X-shaped position on a heating pad of 37°C with tape. The skin of the abdomen was disinfected with 70% ethanol Midline laparotomy was performed by cutting the abdominal skin and muscle in a length of 3 cm to expose the abdominal organs and liver. In the sheet-treated group, the sheet-shaped cell culture obtained in Example 1 was implanted to the liver surface and only a single point was sutured with a suture thread (PDS PLUS (registered trademark), Johnson & Johnson) (Fig. 11B). In contrast, in the control group, suturation was performed with a suture thread to the residual liver in the absence of the sheet-shaped cell culture obtained in Example 1 only at a single point of the site corresponding to the implantation site of the sheet-shaped cell culture in the sheet-treated group. The abdominal cavity and organs were washed with physiological saline and closed with a suture thread. Thereafter, in the sheet-treated group and control group, the skin of the abdomen around the suture thread was disinfected with 70% ethanol. Thereafter, in order to make up for fluid loss due to e.g., bleeding during the surgery, an aseptic isotonic solution (0.5 mL) was subcutaneously injected. Thereafter, TAA intraperitoneal injection to each of the mice twice a week was continued for 3 weeks to induce liver fibrosis to prepare liver-fibrosis model mice (Fig. 11A). The mice were sacrificed on the final day of the 3rd week and the liver was taken out and venous blood was sampled from the tail vein.

Furthermore, similarly to the above, liver-fibrosis model mice (sheet-treated group (n = 5) and control group (n = 5)) (TAA intraperitoneal injection to each of the mice twice a week was continued for 5 weeks to also induce liver fibrosis) were produced together (Fig. 11A). The mice were sacrificed on the final day of the 5th week and the liver was taken out and venous blood was sampled from the tail vein venous blood.

### (2) Histological Examination of Liver

### (2-1) Preparation of Liver Section

The livers obtained in the above (1) were soaked in ethanol for 3 hours, subsequently in xylene for 3 hours, and further in melted paraffin overnight. After solidification by cooling paraffin, sections were prepared using a microtome. The sections were dried and deparaffinized.

### (2-2) Staining with Hematoxylin and Eosin (HE)

The sections obtained in the above (2-1) were stained with a hematoxylin solution (Mayer's hematoxylin, MUTO PURE CHEMICALS CO., LTD.) for 10 minutes. Subsequently, the sections were washed with running water for 15 minutes and stained with an eosin solution (1% eosin Y solution, manufactured by MUTO PURE CHEMICALS CO., LTD.) for 10 minutes. Thereafter, the sections were dewatered with alcohol and naturally dried at room temperature. The sections stained were observed by an optical microscope at 200-fold magnification.

As a result, in the livers taken out from the mice of the sheet-treated group compared to those in the control group, it was confirmed that the area of the fibrosis region decreased (Fig. 12). Thus, it was found that liver fibrosis is suppressed by implantation of the sheet-shaped cell culture to the liver surface.

### (2-3) Sirius Red Staining

The sections obtained in the above (2-1) were stained with a Sirius red solution (1% Sirius red liquid, MUTO PURE CHEMICALS Co., Ltd.) for 10 minutes. Subsequently, the sections were dewatered with alcohol and naturally dried at room temperature. The sections stained were observed by an optical microscope at 200-fold magnification.

As a result, in the livers taken out from the mice of the sheet-treated group compared to those in the control group, it was confirmed that the area of the fibrosis region decreased (Fig. 13). Thus, it was found that liver fibrosis is suppressed by implantation of the sheet-shaped cell culture to the liver surface.

### (2-4) Masson's Trichrome Staining

The sections obtained in the above (2-1) were stained with a Masson's trichrome solution (0.75% orange G solution, 2.5% phosphotungstin acid solution, aniline blue liquid, Masson's staining solution A, MUTO PURE CHEMICALS Co., Ltd.) for 10 minutes. Subsequently, the sections were dewatered with alcohol and naturally dried at room temperature. The sections stained were observed by an optical microscope at 200-fold magnification.

As a result, in the livers taken out from the mice of the sheet-treated group compared to those in the control group, it was confirmed that the area of the fibrosis region decreased (Fig. 14). Thus, it was found that liver fibrosis is suppressed by implantation of the sheet-shaped cell culture to the liver surface.

### (2-5) Immunostaining

The sections obtained in the above (2-1) were washed with phosphate-buffered physiological saline (PBS), and then PBS was removed. The sections were soaked in a 0.5% (v/v) Triton (registered trademark) X-100 solution (penetration treatment solution) dissolved in PBS and incubated for 15 minutes at room temperature. The penetration treatment solution was removed, the sections were washed with PBS, and subsequently, PBS was removed. The sections were soaked in a 3% bovine serum albumin solution (blocking solution) dissolved in PBS and incubated for 60 minutes at room temperature. The blocking solution was removed, the sections were washed with PBS, and subsequently, PBS was removed The sections were soaked in a primary antibody solution (containing anti-Ki67 antibody (ab16667, Abeam)) and incubated for one hour at room temperature. The primary antibody solution was removed, the sections were washed with PBS, and subsequently, PBS was removed. The sections were soaked in a secondary antibody solution (containing an enzyme-labeled antibody (LSAB2 System-HRP (K0672), Agilent Technologies)) and incubated for 30 minutes at room temperature. The secondary antibody solution was removed, the sections were washed with PBS, and subsequently, PBS was removed. The sections were soaked in an enzyme-substrate solution (containing an enzyme substrate (LSAB2 System-HRP (K0672), Agilent Technologies)) and incubated for 30 minutes at room temperature to produce a color reaction. The enzyme-substrate solution was removed, the sections were washed with PBS, and subsequently, PBS was removed. The sections stained were observed by an optical microscope.

As a result, in the livers excised from mice of the sheet-treated group compared to those taken from the control group, the number of Ki67 (cell proliferation marker)-positive cells was higher (Fig. 15). Thus, it was found that the proliferation of liver cells and regeneration of the liver tissue are promoted by implantation of the sheet-shaped cell culture to the liver surface.

### (3) Clinical Chemistry Test for the Liver

### (3-1) Preparation of Lysate

The livers obtained in the above (1) were washed with cooled PBS and cut into small pieces while they were cooled on the ice. These pieces were put in a homogenizer and RIPA buffer (containing a protease inhibitor) was added thereto. The mixture was homogenized, ultrasonically treated, and then centrifuged at 10,000 × g and 4°C for 20 minutes to obtain the supernatant (lysate).

### (3-2) Preparation of serum from venous blood

The venous blood obtained in the above (1) was centrifuged at 10,000 × g and 4 °C for 10 minutes to obtain a supernatant (serum).

### (3-3) Real-time PCR

Total RNA was purified from lysates obtained in the above (3-1) using a commercially available RNA purification kit (Rneasy Mini Kit, QIAGEN). Thereafter, the RNA was subjected to RNA reverse transcription and real-time PCR using a commercially available real-time PCR kit (Reverse Transcription System, Promega) to obtain the expression levels of mRNA of αSMA and Collagen type I α1 contained in the lysate obtained in the above (3-1). For αSMA, the forward primer was represented by 5'-GTCCCAGACATCAGGGAGTAA-3' (SEQ ID NO: 5) and the reverse primer was represented by 5'-TCGGATACTTCAGCGTCAGGA-3' (SEQ ID NO: 6). Furthermore, for Collagen type I α1, the forward primer was represented by 5'-GCTCCTCTTAGGGGCCACT-3' (SEQ ID NO: 7), the reverse primer was represented by 5'-CCACGTCTCACCATTGGGG-3' (SEQ ID NO: 8). The thermal cycler settings were as shown in Table 2.

**Table 2. Thermal cycler settings**

| [Table 2] | | |
|---|---|---|
| Temperature | Time | Number of cycles |
| 98°C | 1 minute | - |
| 98°C | 10 seconds | 30 times |
| 55°C | 15 seconds | |
| 72°C | 40 seconds | |
| 72°C | 2 minutes | - |

As a result, in the livers excised from the mice of the sheet-treated group compared to those in the control group, it was confirmed that the expression levels of mRNA of αSMA (liver fibrosis marker) and Collagen type I α1 (liver fibrosis marker) are lower (Fig. 16). Thus, it was found that liver fibrosis is suppressed by implantation of the sheet-shaped cell culture to the liver surface.

### (3-4) ELISA

The serum obtained in the above (3-2) was subjected to ELISA in Oriental Yeast Co., Ltd. and the expression levels of total protein, albumin, aspartate aminotransferase (AST), alanine aminotransferase (ALT), and total bilirubin contained in the serum were quantified.

As a result, in livers excised from the mice of the sheet-treated group compared to those in the control group, it was confirmed that the expression level of the total protein is higher at 3 weeks and 5 weeks after surgery; the expression level of albumin is lower at 3 weeks after surgery but higher at 5 weeks after surgery; the expression level of AST is lower at 3 weeks and 5 weeks after surgery; the expression level of ALT is higher at 3 weeks after surgery but lower at 5 weeks after surgery; and the expression level of total bilirubin is lower at 3 weeks and 5 weeks after surgery (Fig. 17). Thus, it was found that liver dysfunction can be suppressed by implanting the sheet-shaped cell culture to the liver surface.

From the results in the foregoing, according to the present invention, liver dysfunction such as hepatitis, liver fibrosis, liver cirrhosis, liver cancer, or liver failure can be treated. In particular, according to the present invention, it is possible to treat terminal-stage decompensated cirrhosis whose improvement cannot be expected by current internal therapy as well as to treat liver failure of residual liver after liver cancer resection. Furthermore, according to the present invention, it is possible to promote proliferation of the liver cells, regeneration of the liver tissue and/or angiogenesis. Furthermore, according to the present invention, it is possible to suppress liver fibrosis. Moreover, according to the present invention, it is possible to treat liver dysfunction through cytokine production.

## Claims

1. A sheet-shaped cell culture comprising skeletal myoblasts for treating liver dysfunction or improving liver function.

2. The sheet-shaped cell culture according to claim 1, wherein the liver dysfunction is hepatitis, liver fibrosis, liver cirrhosis, liver cancer, or liver failure.

3. The sheet-shaped cell culture according to claim 1 or 2, wherein the liver dysfunction is liver cirrhosis.

4. The sheet-shaped cell culture according to claim 1 or 2, wherein the liver dysfunction is liver cancer.

5. The sheet-shaped cell culture according to claim 4, for application to the liver after liver cancer resection.

6. The sheet-shaped cell culture according to any one of claims 1 to 5, for promoting proliferation of the liver cells, regeneration of a liver tissue and/or angiogenesis.

7. The sheet-shaped cell culture according to any one of claims 1 to 6, for suppressing liver fibrosis.

8. The sheet-shaped cell culture of any one of claims 1 to 7, for treating liver dysfunction via cytokine production.

9. The sheet-shaped cell culture according to any one of claims 1 to 8, further comprising a reinforcement layer containing a gel and/or a polymer.

10. A method for producing the sheet-shaped cell culture according to any one of claims 1 to 9, the method comprising:
seeding a cell population containing skeletal myoblasts on a culture substrate;
forming a sheet of the cell population seeded to form a sheet-shaped cell culture; and
detaching the formed sheet-shaped cell culture from the culture substrate.

11. A method for treating liver dysfunction, the method comprising applying the sheet-shaped cell culture according to any one of claims 1 to 9 to a site exhibiting the liver dysfunction.

12. The method of claim 11, wherein the step of applying the sheet-shaped cell culture to a site exhibiting liver dysfunction is performed by implanting the sheet-shaped cell culture onto a liver surface with an implantation device.
